# EUROPEAN PATENT APPLICATION

(11) **EP 4 272 772 A1**
(43) Date of publication of application: **08.11.2023**
(21) Application number: 21914435.9
(22) Date of filing: 28.12.2021
(51) Int. Cl.: A61L 27/36, A61L 27/54, A61L 27/00

(54) **PRO-ENDOTHELIALIZATION BIOMATERIAL, HEART VALVE, AND PREPARATION AND APPLICATION THEREOF**

(30) Priority: 31.12.2020 CN 202011626626
(71) Applicant: Venus MedTech (HangZhou), Inc., Hangzhou, Zhejiang 310052 (CN)
(72) Inventor: LIU, Zhaogang, Hangzhou, Zhejiang 310052 (CN); ZHU, Yuting, Hangzhou, Zhejiang 310052 (CN); KUANG, Dajun, Hangzhou, Zhejiang 310052 (CN); LIM, Hou-Sen, Hangzhou, Zhejiang 310052 (CN)
(74) Representative: KIPA AB
(86) International application number: PCT/CN2021/142207
(87) International publication number: WO 2022/143701

(57) **Abstract**

Provided are a pro-endothelialization biological material and method for preparation thereof, and a pro-endothelialization heart valve and method for preparation thereof and interventional system. The pro-endothelialization biological material comprises a biological material and a pro-endothelializing growth factor loaded on the surface of biological material; capable of capturing endothelial progenitor cells, enabling the endothelial progenitor cells to attach, grow, and differentiate on the surface of the biological material. The methods for preparation of the pro-endothelialization biological material comprises chemical grafting and physical coating methods. The heart valve can be prepared either by suturing followed by grafting or by grafting followed by suturing. A endothelial growth factor is directly loaded onto the surface of a biological material film, promoting the formation of endothelial cell layers on the surface of the biological material.

## Description

### TECHNICAL FIELD

The present disclosure relates to the technical field of implantable biological materials, in particular to a pro-endothelialization biological material, a heart valve, and preparation and application thereof.

### DESCRIPTION OF THE PRIOR ART

In recent years, interventional biological material (such as interventional valve) replacement surgery has a wide clinical application due to its convenient operation and reducing the secondary damage to patients greatly. By treating the surface of the interventional valve, it can capture endothelial progenitor cells after being implanted in the human body, promoting the endothelialization of the valve, enhancing the biocompatibility of the valve and prolonging the service life of the valve. The heart valve includes a stent and a valve provided in the stent. In existing techniques, the capture factor for capturing the endothelial progenitor cells is loaded on the stent, with a poor loading stability. To solve this problem, excess crosslinking agent must be used.

### SUMMARY OF THE DISCLOSURE

The present disclosure provides a pro-endothelialization heart valve and preparation and application thereof. By directly chemically or physically loading endothelialization growth factors on the surface of the valve, there is no need to use additional crosslinking agents.

A pro-endothelialization biological material is provided, and the surface of the biological material is loaded with an endothelial growth factor.

The endothelial growth factor refers to a species that can capture endothelial progenitor cells to allow endothelial progenitor cells to adhere, grow, and differentiate on the surface of the implanted biological material, so as to induce generation of a functional endothelial cell layer on the surface of the implanted biological material.

Optionally, the endothelial growth factor has a loading amount of: 0.1 to 20 µg of the endothelial growth factor on a surface of the biological material per surface area of 1 cm².

Optionally, the biological material is a glutaraldehyde cross-linked biological material.

The glutaraldehyde cross-linked biological material is a biological tissue cross-linked by glutaraldehyde; the biological material is originated from pig or cattle, and selected from pericardium, heart valve, blood vessel, ligament, muscle, intestine or skin. These biological tissues are rich in collagen fibers, which are rich in amino groups and carboxyl groups. A cross-linked network is formed through the reaction of the aldehyde groups of the glutaraldehyde with the amino groups or carboxyl groups.

Optionally, the endothelial growth factor is CD34 antibody, CD133 antibody or vascular endothelial growth factor VEGF.

The CD34 antibody is loaded on the surface of the biological material. By means of the specific binding of the CD34 antibody to the CD34 antigen on the surface of the endothelial progenitor cell, the endothelial progenitor cell is captured to adhere, grow, and differentiate on the surface of the biological material and form a functional endothelial cell layer. The CD34 antibody here is mouse anti-human CD34 antibody, KG-1a type. The antibody can be obtained by cloning methods well known in the art, or be obtained commercially. The same applies to CD133 antibody and VEGF growth factor.

Optionally, the endothelial growth factor is loaded on a surface of the biological material by chemical grafting or physical coating.

A preparation method for a pro-endothelialization biological material is provided, including:

exposing a glutaraldehyde cross-linked biological material after removing free glutaraldehyde into a solution containing an endothelial growth factor for reaction, wherein the solution has a pH value of neutrality or weak alkalinity.

In the glutaraldehyde cross-linked biological material, two ends of some glutaraldehydes are connected with the amino groups or carboxyl groups of the collagen fibers, respectively, and cross-linked in the network, and one ends of some glutaraldehydes are connected with the amino groups or carboxyl groups of the collagen fibers, the other ends are in a free state, i.e., residual aldehyde groups, remaining free glutaraldehydes that has not participated in the reaction. The remaining free glutaraldehydes needs to be removed before coating the endothelial growth factor, thereby avoiding possible disruption on the activity of the endothelial growth factor.

The treatment method is a chemical grafting method. The chemical grafting mechanism is: CD34 antibody or CD133 antibody or VEGF growth factor contains amino functional groups, and the biological material contains residual aldehyde groups. Under a neutrality or weak alkalinity condition, a chemical reaction occurs between the amino functional groups of the antibody and the aldehyde groups on the valve, thereby grafting the antibody onto the surface of the valve material.

Optionally, in the solution containing the endothelial growth factor, the endothelial growth factor has a concentration ranging from 1 to 50 ug/ml.

Further optionally, the solution containing the endothelialization growth factor further contains a polyamino substance.

Optionally, the polyamino substance is at least one of polyethylene glycol ammonia, aminopropylene glycol, amino acid and polypeptide.

Optionally, the polyamino substance has a concentration ranging from 1 to 50 ug/ml.

When the concentration of the endothelial growth factor is small and the grafting amount of the endothelial growth factor is not enough to react all residual aldehyde groups, the added polyamino substance will compete for the residual aldehyde groups on the surface of the material, eliminating the impact of the residual aldehyde groups on the activity of the endothelial growth factor.

Optionally, the solution containing the endothelial growth factor contains a solvent being PBS buffer, phosphate buffer or Tris-hydrochloric acid buffer, with a pH value ranging from 7 to 8.

Optionally, the method includes stopping the reaction when the endothelial growth factor on a surface of the pro-endothelialization biological material reaches a content ranging from 0.1 to 20 ug/cm².

Optionally, the reaction time ranges from 1 to 6 hours. When the concentration of the endothelial growth factor ranges from 1 to 50 ug/ml, the content of the endothelial growth factor on the surface of the pro-endothelialization biological material can reach 0.1 to 20 ug/cm² after reacting for 1 to 6 h.

The chemical grafting reaction is carried out at normal room temperature, and there is no need to deliberately control the reaction temperature. Optionally, the temperature of the reaction ranges from 0 to 37 °C.

Optionally, during the reaction, the glutaraldehyde cross-linked biological material is in dynamic contact or static contact with the solution containing the endothelial growth factor.

During the dynamic contact, the glutaraldehyde cross-linked biological material and the solution containing the endothelial growth factor are movable relative to each other, which can be realized by spraying repeatedly, or stirring or shaking the reaction system during the reaction, for example, by soaking the glutaraldehyde cross-linked biological material in the reaction solution and then placing the same in a shaker for reaction. During the static contact, the glutaraldehyde cross-linked biological material and the solution containing the endothelial growth factor are relatively static, for example, the glutaraldehyde cross-linked valve can be directly soaked in the reaction solution for static reaction. The static contact or dynamic contact mentioned below can be explained in the same way unless otherwise specified.

Optionally, the preparation method further includes: exposing the glutaraldehyde cross-linked biological material after the reaction into a softening agent solution.

For the biological material grafted with antibody, the softening agent should not destroy the activity of the antibody during the softening treatment. Optionally, the softening agent solution is a mixed solution of glycerin and polyethylene glycol or pure glycerin; and the glycerin has a volume percentage ranging from 20% to 100%. When the volume percentage of the glycerin is 100%, it is pure glycerin. When the volume percentage of glycerin is less than 100%, it is a mixed solution of glycerin and polyethylene glycol.

The glycerin solution is formulated by glycerin (20% to 100%) + PEG. After soaking in the glycerin solution for 0.5 to 4 hours, the material is then taken out and the free glycerin is removed, and then the material is dehydrated or dried, and preserved in a dry condition. Alternatively, the material can be directly soaked in the glycerin solution for preservation.

Alternatively, the softening agent can be a mixed solution of glycerin and water; a mixed solution of glycerin and alcohol; a mixed solution of glycerin, water and alcohol; polyethylene glycol; a mixed solution of polyethylene glycol and water; a mixed solution of polyethylene glycol and alcohol; or a mixed solution of polyethylene glycol, water and alcohol.

In the case with glycerin, the volume percentage of glycerin ranges from 20% to 100%; in the case with polyethylene glycol, the volume percentage of polyethylene glycol ranges from 20% to 100%.

Optionally, the preparation method further includes: dehydrating or drying the glutaraldehyde cross-linked biological material treated with the softening agent after removing free glycerin.

Dehydration is an optional and preferred step. Preservation after dehydration is more conducive to keep the antibody activity. Alternatively, the material can be directly soaked in the corresponding solution for preservation after softening treatment. The material should be preserved in an inert condition without affecting the CD34 antibody activity, such as in a pure glycerin solution, a mixed solution of glycerin and water, a mixed solution of glycerin and alcohol, a pure polyethylene glycol solution, a mixed solution of polyethylene glycol and water, a mixed solution of polyethylene glycol and alcohol among others. The preservation temperature can be low temperature or normal temperature, preferably at -10 to 25 °C.

A preparation method for a pro-endothelialization biological material is provided, including: exposing a glutaraldehyde cross-linked biological material after removing free glutaraldehyde into a solution containing a capping substance and a solution containing an endothelial growth factor in sequence.

The capping substance is a substance that blocks residual aldehyde groups in the glutaraldehyde cross-linked biological material, and is used to block the residual aldehyde groups of the glutaraldehyde cross-linked biological material to eliminate the impact of the residual aldehyde groups on the activity of the endothelial growth factor. This treatment method is a physical grafting method, and the physical grafting mechanism is: first blocking the residual aldehyde groups on the surface of the biological material, and then coating the matrix solution dissolved with the endothelialization growth factor on the surface of the biological material.

Optionally, the capping substance contains at least one group capable of reacting with the residual aldehyde group in the glutaraldehyde cross-linked biological material.

Optionally, the group for reacting with the residual aldehyde group is an amino group; and the solution containing the capping substance has a pH value of neutrality or weak alkalinity.

Optionally, the capping substance is a polyamino substance.

Optionally, the capping substance is at least one of polyethylene glycol ammonia, aminopropylene glycol, amino acid and polypeptide.

Optionally, in the solution containing the capping substance, the capping substance has a mass percent concentration ranging from 0.1% to 5%.

Optionally, the solution containing the capping substance contains a solvent being PBS buffer, phosphate buffer or Tris-hydrochloric acid buffer, with a pH value ranging from 7 to 8.

Optionally, the method includes stopping exposing the glutaraldehyde cross-linked biological material in the solution containing the capping substance when the residual aldehyde group amount of the glutaraldehyde cross-linked biological material is below a detectable value.

Below a detectable value means that the residual aldehyde groups cannot be detected by conventional detection methods, and in this case, it can also be considered that all the residual aldehyde groups are completely blocked.

Optionally, the exposure time is 1 to 6 h. When the mass percent concentration of the capping substance is 0.1% to 0.5% as mentioned above, the contact reaction can be carried out for 1 to 6 hours to completely block the residual aldehyde groups.

When treating the residual aldehyde groups, in addition to using amino-containing substances to directly react with the residual aldehyde groups through their amino groups, a reductant can alternatively be used to reduce the residual aldehyde groups to hydroxyl groups, so as to eliminate the impact of the residual aldehyde groups on the activity of the endothelial growth factor. The reductant can be sodium borohydride or potassium borohydride.

After removing the residual aldehyde groups as described above, the biological material is exposed into a solution containing the endothelial growth factor, and the endothelial growth factor is grafted onto the surface of the biological material by a physical coating method.

Optionally, the endothelial growth factor is CD34 antibody, CD133 antibody or vascular endothelial growth factor VEGF.

Optionally, a concentration of the endothelial growth factor in the solution containing the endothelial growth factor and the exposure time in the solution containing the endothelial growth factor are selected such that after finishing the preparation, the endothelial growth factor on a surface of the glutaraldehyde cross-linked biological material reaches a content ranging from 0.1 to 20 ug/cm².

Optionally, the solution containing the endothelial growth factor contains a solvent being PBS buffer, phosphate buffer or Tris-hydrochloric acid buffer; and the endothelial growth factor has a concentration ranging from 1 to 50 ug/ml.

Optionally, within the above concentration range, the exposure time in the solution containing the endothelial growth factor is 1 to 6 hours.

The chemical grafting reaction is carried out at normal room temperature, and there is no need to deliberately control the reaction temperature. Optionally, the reaction temperature in the solution containing the endothelial growth factor is 0 to 37 °C.

Optionally, the solution containing the endothelial growth factor has a pH value of neutrality.

Optionally, the method includes exposing the glutaraldehyde cross-linked biological material into the solution containing the capping substance and the solution containing the endothelial growth factor by dynamic contact or static contact.

Optionally, the method further includes exposing the glutaraldehyde cross-linked biological material after the reaction into a softening agent solution.

In the physical coating method, the softening treatment step, drying and dehydration step and preservation condition are all the same as that in the chemical grafting method.

A pro-endothelialization biological material prepared by the preparation method above is provided.

The biological tissue prepared according to the present disclosure can be used for interventional biological valve, for example, through minimally invasive intervention, and can alternatively be used for surgical biological valve, for example, being implanted through surgery.

A pro-endothelialization heart valve includes a cylindrical stent and a valve provided in the stent, and the valve is the pro-endothelialization biological material according to the present disclosure.

A preparation method for a pro-endothelialization heart valve is provided. The heart valve includes a stent and a valve sewn on the stent, and the heart valve is a glutaraldehyde cross-linked biological material. The heart valve is treated with endothelial growth factor grafting according to the method (chemical grafting or physical coating) as described above.

A preparation method for a pro-endothelialization heart valve is provided. The heart valve includes a stent and a valve, and the valve is a glutaraldehyde cross-linked biological material. The valve is sewn on the stent after being treated with endothelial growth factor loading (chemical grafting or physical coating) as described above.

A pro-endothelialization heart valve prepared by the preparation method above is provided.

The pro-endothelialization heart valve according to the present disclosure can be a valve through minimally invasive intervention, or being implantable through surgery.

An interventional system includes a heart valve and a delivery catheter, the heart valve is configured to be delivered by the delivery catheter, and the heart valve is the pro-endothelialization heart valve as described above.

Compared with the prior arts, the present invention has at least one of the following beneficial effects:
(1) The antibody used in the present disclosure has high activity in capturing endothelial progenitor cells, with high specificity and capture ability, as well as a faster endothelialization speed.
(2) In the chemical grafting method according to the present disclosure, there is no need to use additional crosslinking agents, and a large amount of antibodies are grafted on the surface of the cross-linked valve through the residual aldehyde groups, which reduces costs and pollution caused by waste liquid.
(3) In the chemical grafting method according to the present disclosure, the residual aldehyde groups on the surface of the biological material is directly used as the linking groups to react with the amino groups of the endothelial growth factor, which eliminates the possible impact of the residual aldehyde groups on the activity of the endothelial growth factor, and also omits the step of treating the residual aldehyde groups.
(4) In the physical coating method according to the present disclosure, it is only necessary to block the residual aldehyde groups with a capping substance or a reductant, and then perform the coating in one step.
(5) According to the present disclosure, both the chemical grafting method and the physical coating method directly combine the endothelial growth factor on the surface of the biological material, and the endothelial cells are directly coated on the surface of the biological material. After the material is implanted in the human body, a long-term endothelialization on the valve surface can be maintained, avoiding the risk of falling of the substance adhered to the surface of the matrix.

### BRIEF DESCRIPTION OF DRAWINGS

FIG. 1 is a flow chart according to the present disclosure.
FIG. 2 is a graph showing the results of grafting uniformity of valve materials according to the present disclosure.
FIG. 3 is a graph showing the results of adhesion to platelets of valve materials according to the present disclosure.
FIG. 4 is a graph showing the results of adhesion to in vitro cells of valve materials according to the present disclosure.
FIG. 5 is a graph showing the results of in vitro cell proliferation of valve materials according to the present disclosure.
FIG. 6 is a graph showing the results of washing resistance of the antibody coating of valve materials according to the present disclosure.
FIG. 7 is a graph showing the results of long-term stability of the antibody coating of valve materials according to the present disclosure.

### DDESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this invention.

Unless otherwise defined, all technical and scientific terms used herein have the same meaning as commonly understood by a person skilled in the art. The terms in the description of the present disclosure are used to describe specific embodiments, and not to limit the present disclosure.

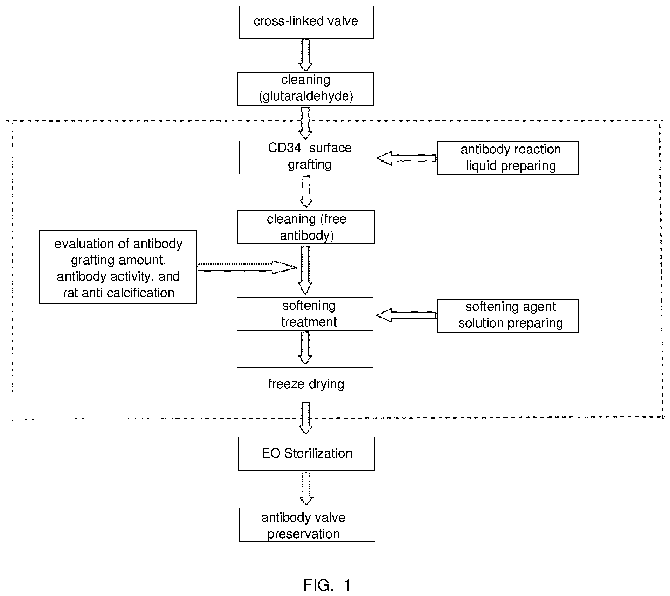

After cleaning the free glutaraldehyde on the surface of the glutaraldehyde cross-linked biological material, the surface of the glutaraldehyde cross-linked biological material is grafted with endothelial growth factor. After grafting, the free endothelial growth factor is removed. The biological material is then subjected to softening treatment, sterilization and preserved by appropriate methods. Taking grafting CD34 antibody as an example, the flow is generally shown in FIG. 1. The steps of washing free antibodies, sterilization and preserving antibodies shown in the figure are all routine technique and are not intended to limit the method of the present invention.

**Surface grafting:** surface contact method uses chemical grafting and physical coating.

Chemical grafting: after removing the free glutaraldehyde, soaking the cross-linked biological material into a solution containing endothelial growth factor, and reacting under a neutrality or weak alkalinity condition.

Physical coating: after removing the free glutaraldehyde, eliminating the aldehyde groups of the cross-linked biological material using an amino-containing reagent under a neutrality or weak alkalinity condition, and soaking the treated valve in a neutral solution containing endothelial growth factor for coating.

Amino reagents for eliminating the aldehyde groups include: polyethylene glycol ammonia, aminopropylene glycol, amino acid, and polypeptide.

In addition to using amino reagents to eliminate aldehyde groups, reductant can be alternatively used. The reductant can be sodium borohydride or potassium borohydride. The concentration of the reductant solution is 0.1% to 2%, and the solvent is water, ethanol, methanol or in combination, with an alcohol content of 0 to 100%. The reaction continues for 0.5 to 4 h at room temperature.

For the grafting step, chemical cross-linking method (under UV radiation, N-hydroxysuccinimide group (NHS) at one end of a crosslinking agent SANPAH reacts with the amino group of the CD34 antibody, thereby binding the CD antibody to SANPAH) and layer-by-layer assembly method (immobilizing negatively charged antibody molecule CD34 to the surface of polyelectrolyte with polycation as the outermost layer) are performed as contrast experiments.

**Softening treatment:** soaking the biological material after surface contact treatment in a softening agent solution for softening treatment.

**Dehydration treatment:** using freeze drying or ethanol dehydration.

Sterilization and preservation: sterilizing the dry biological material after dehydration and preserving the same.

Sterilization method: ethylene oxide or radiation sterilization.

The specific examples are described below:

The antibodies used in the following examples are selected from mouse anti-human CD34 antibody, KG-1a type, and animal-derived clonal antibodies, which can be obtained by conventional cloning methods or commercially available. The antibodies used in the following examples are obtained commercially.

### Example 1 Chemical grafting

A sewn heart valve was washed with physiological saline to remove the free glutaraldehyde, and then the heart valve containing residual aldehyde groups was soaked in a CD34 antibody solution, which is a PBS solution containing 20 ug/ml CD34 antibody with a pH value of 7.4. Then, the antibody solution containing the valve was placed on a shaker and shaken for 2 hours with the temperature controlled at 20 °C. The valve was then taken out and placed in pure glycerin solution and soaked at room temperature for 1 h. Excess glycerol was removed from the surface of the valve, rapid centrifugation was performed at 1000 r/min for 5 minutes, thereby finishing the preparation and obtaining a heart valve with CD34 antibody immobilized on the surface thereof. The valve was then packaged and sterilized with ethylene oxide.

### Example 2 Chemical grafting

A sewn heart valve was washed with physiological saline to remove the free glutaraldehyde, and then the heart valve containing residual aldehyde groups was soaked in a CD34 antibody solution, which is a PBS solution containing 5 ug/ml CD34 antibody and 50 ug/ml polyethylene glycol ammonia with a pH value of 7.4. Then, the antibody solution containing the valve was placed on a shaker and shaken for 2 hours with the temperature controlled at 20 °C. The valve was then taken out and placed in pure glycerin solution and soaked at room temperature for 1 h. Excess glycerol was removed from the surface of the valve, and rapid centrifugation was performed at 2000 r/min for 5 minutes. The valve was then packaged and sterilized with radiation.

### Embodiment 3 Chemical grafting

A sewn heart valve was washed with physiological saline to remove the free glutaraldehyde, and then the heart valve containing residual aldehyde groups was soaked in a CD34 antibody solution, which is a borate solution containing 10 ug/ml CD34 antibody with a pH value of 7.4. Then, the antibody solution containing the valve was placed on a shaker and shaken for 1 h with the temperature controlled at 20 °C. The valve was then taken out and placed in a mixed solution of glycerin and ethanol and soaked at room temperature for 1 h. Excess glycerol was removed from the surface of the valve, rapid centrifugation was performed at 1000 r/min for 10 minutes, thereby finishing the preparation and obtaining a heart valve with CD34 antibody immobilized on the surface thereof. The valve was then packaged and sterilized with ethylene oxide.

### Example 4 Physical coating

A sewn heart valve was washed with physiological saline to remove the free glutaraldehyde, and then the heart valve containing residual aldehyde groups was soaked in a PBS solution containing 0.1 wt% polyethylene glycol ammonia with a pH value of 7.4. Then, the solution containing the valve was placed on a shaker and shaken for 4 hours with the temperature controlled at 37 °C. The valve was then placed in a solution containing 10 ug/ml CD34 antibody and soaked for 0.5 h at room temperature. Then the valve was soaked in pure glycerin solution for 2 hours, taken out, and subjected to rapid centrifugation at 1000 r/min for 2 minutes, thereby finishing the preparation and obtaining a heart valve with CD34 antibody immobilized on the surface thereof. The valve was then packaged and sterilized with ethylene oxide.

### Example 5 Grafting and then sewing

A glutaraldehyde cross-linked valve was washed with physiological saline to remove the free glutaraldehyde, and then the cross-linked valve containing residual aldehyde groups was soaked in a CD34 antibody solution, which is a PBS solution containing 5 ug/ml CD34 antibody and 50 ug/ml polyethylene glycol ammonia with a pH value of 7.4. Then the antibody solution containing the cross-linked valve was placed on a shaker and shaken for 2 hours with the temperature controlled at 20 °C. The cross-linked valve was taken out, cut, and sewn to a metal stent. The sewn heart valve was placed in pure glycerin solution at room temperature for 1 h. Excess glycerol was removed from the surface of the valve, and rapid centrifugation was performed at 1000 r/min for 5 minutes. The valve was then packaged and sterilized with ethylene oxide.

### Example 6 Reduction and then physical coating

A solution in which the concentration of sodium borohydride is 0.2%, the solvent is a mixed solution of water and ethanol, and the ethanol content is 50%, was prepared. Then the sewn heart valve is washed with physiological saline to remove the free glutaraldehyde, placed in the above-prepared solution, and soaked at room temperature for 2 hours. Then the valve was washed with physiological saline, placed in a solution containing 10 ug/ml CD34 antibody and soaked at room temperature for 2 h, and then soaked in pure glycerin solution for 2 hours. The valve was taken out, and subjected to rapid centrifugation at 1000 r/min for 1 minute, thereby finishing the preparation and obtaining a heart valve with CD34 antibody immobilized on the surface thereof. The valve was then packaged and sterilized with ethylene oxide.

### Control group 1

A reaction mixture of CD34 antibody and SANPAH (6-[(4-azido-2-nitrophenyl)amino] succinimidyl hexanoate) was prepared: with the chemical crosslinking agent SANPAH as medium, absolute ethanol was titrated with 4% NaOH to adjust the pH value to 7.9, thereby preparing a 0.133 mmol/L SANPAH solution in the dark. The solution was added into an antibody solution, with CD34 antibody and SANPAH at a molar ratio of 1 : 20, mixed and reacted at room temperature in the dark for 2 hours, and centrifuged at 10,000 xg for 10 minutes to remove the unreacted antibody and SANPAH.

### Control group 2

The cross-linked valve was placed in 20 ug/ml anti-CD34 antibody solution (in which there is a cationic compound such as chitosan) and acted at 4 °C for 12 hours to obtain an anti-CD34 antibody-modified valve.

### Grafting uniformity

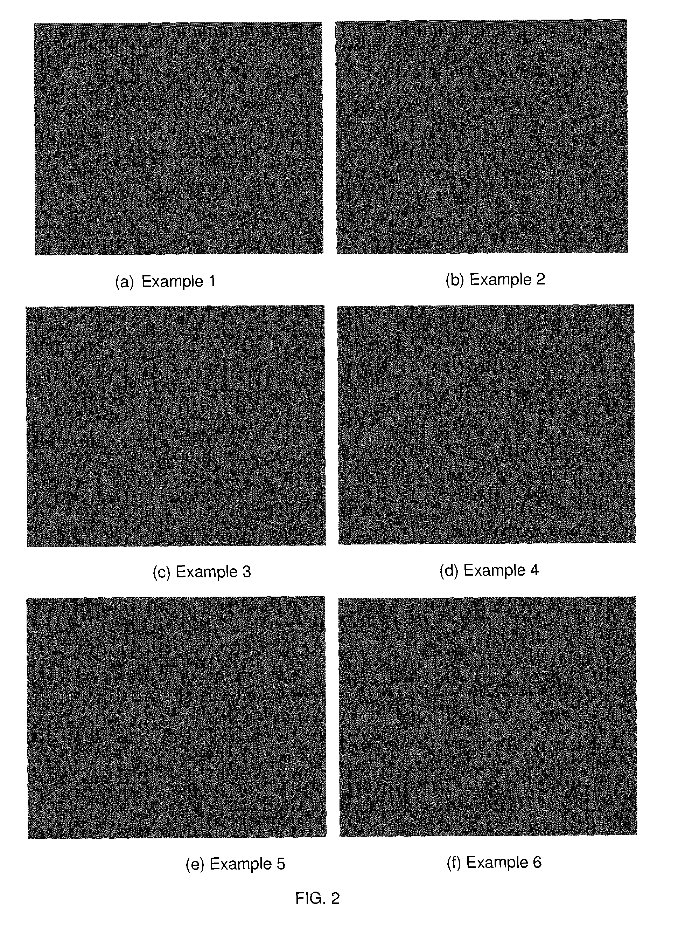

The uniformity of fluorescence intensity distribution on the surface of the valve was observed by immunofluorescence to characterize the grafting uniformity on the surface of the valve. Specifically, (1) valves (prepared according to Example 1 to Example 6, respectively) immobilized with CD34 antibody were rinsed three times with PBS at room temperature, each time for 5 minutes; (2) fluorescent secondary antibody (rabbit anti-mouse IgG marked with PE) was added to the rinsed valves at a molar ratio of 1 : 400, incubated at room temperature for 1 hour in the dark; (3) the unreacted fluorescent secondary antibody was removed, and the valves were rinsed with PBS at room temperature three times, each time for 10 minutes; (4) the treated valves were fixed on glass slides and sent for immunofluorescence detection. The detection results shown in FIG.2 show that CD34 antibody can be detected in all of the examples, indicating the method according to the present disclosure has a good grafting uniformity.

### Grafting amount and antibody activity

### Enzyme-Linked Immunosorbnent Assay (ELISA) was used to detect the amount of CD34 antibody immobilized on the surface of the valve. Specifically, (1) the valves immobilized with the antibody according to Example 1 to Example 6 were placed in a 24-orifice plate, and blocked with 10% BSA for 1 hour at 37 °C, immersed and cleaned in washing liquid (physiological saline) for 3 * 5 min; (directly immobilized BSA on the wet valve surface and directly immobilized BSA on the dry valve surface were used as negative controls); (2) added with HRP-IgG at 100 ul/orifice for 1 hour at 37 °C, immersed and cleaned in washing liquid (physiological saline) for 3 * 5 min; (3) added with TMB chromogenic solution at 100 ul/orifice for 30 min at 37 °C; (4) added with TMB stop solution at 50 ul/orifice; (5) the OD (450nm) value of the obtained solution was measured with a microplate reader.

The test results are shown in Table 1, which shows that the range of the amount of CD34 antibody on the surface of the valve prepared according to Examples 1 to 6 is: the surface of the biological material contains 9.86 to 18.94 µg of CD34 antibody per surface area of 1 cm².

**Table 1**

| Example No. | Antibody content per surface area / (µg/cm²) |
|---|---|
| Example 1 | 12.85±1.01 |
| Example 2 | 10.77±1.62 |
| Example 3 | 9.86±0.82 |
| Example 4 | 18.72±1.98 |
| Example 5 | 15.88±1.18 |
| Example 6 | 18.94±1.79 |

### Adhesion to platelets

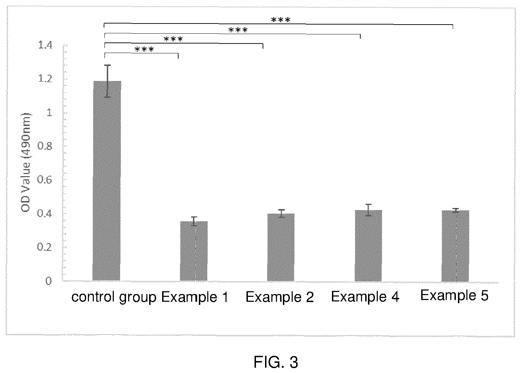

The content of lactate dehydrogenase (LDH) released from platelets adsorbed on the valve materials was determined by testing kit to detect the adhesion of the valve materials to platelets. Specifically, (1) after the sample valves (prepared according to Examples 1, 2, 4, and 5, with a glutaraldehyde cross-linked valve unmodified with CD34 as control group) reacted with fresh platelet-rich plasma at 37 °C for 30 minutes, the valves were carefully washed with PBS and fixed with 1% glutaraldehyde solution for 30 min; (2) washed with distilled water, and then dehydrated stepwise with ethanol water gradient solution (30, 40, 50, 60, 70, 80, 90, 95, 100% v/v); (3) after natural drying in the air, 150 µL LDH release reagent diluted ten times with PBS was added to a 96-orifice plate and the orifice plate was shaken to mix, and then incubated in an oven at 37 °C for one hour; (4) 120 µL supernatant from each orifice was taken out and added to a new 96-orifice plate, and then a detection solution containing three solutions (lactic acid solution, enzyme solution and 1X INT solution) with volume ratio of 1:1:1 was prepared. 60 µL detection solution was added to the supernatant of each orifice, mixed, and incubated on a horizontal shaker at room temperature in the dark for 25 min. Then the absorbance of the solution at 490 nm was detected with a microplate reader. The test results are shown in FIG. 3, which shows that the cross-linked valves modified with CD34 have good anti-platelet adhesion.

### Adhesion to in vitro cells

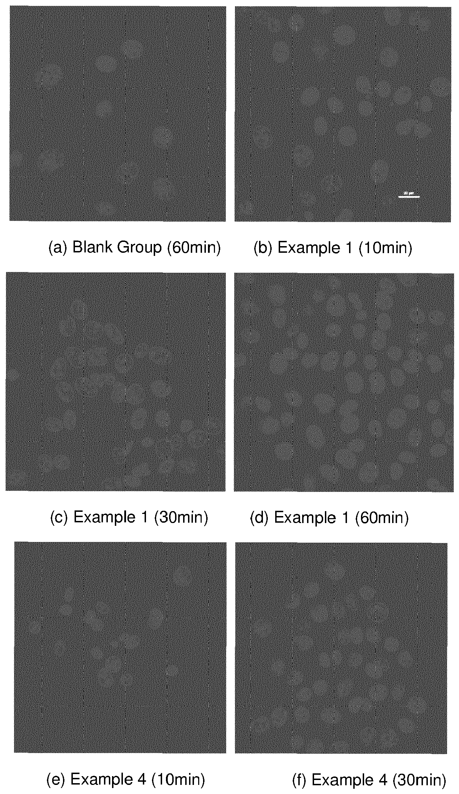

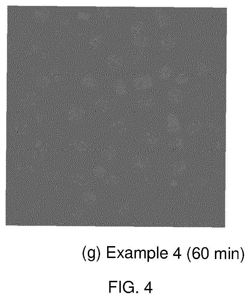

Fluorescence microscopy or flow cytometry was used to observe and record the number of cells on the surface of the valves. Specifically, endothelial cells were incubated at a density of 1.5 * 10⁴ cell/orifice into a 24-orifice cell culture plate with sample valves (prepared according to Examples 1 and 4, with a glutaraldehyde cross-linked valve unmodified with CD34 as control group) at the bottom. After incubating for 10 min, 30 min, and 60 min respectively, the original culture solution was discarded, and PBS was used to wash the valves for 3 times. After FDA staining for 15 min, washing with PBS 5 times, a fluorescent microscope was used to take pictures for observation. imaged software was used to count the cells on the pictures. The valve with unmodified CD34 antibody was used as a contrast experiment. Alternatively, after the cells have been cultured for a period of time, they are digested with trypsin and counted manually under a phase-contrast microscope with a hemocytometer. Alternatively, a flow cytometry was used to measure the number of cells on the valve surface. The test results are shown in FIG. 4, which shows that the cross-linked valves modified by CD34 have good adhesion to endothelial cells, which are significantly better than that of the unmodified cross-linked valve.

### In vitro cell proliferation

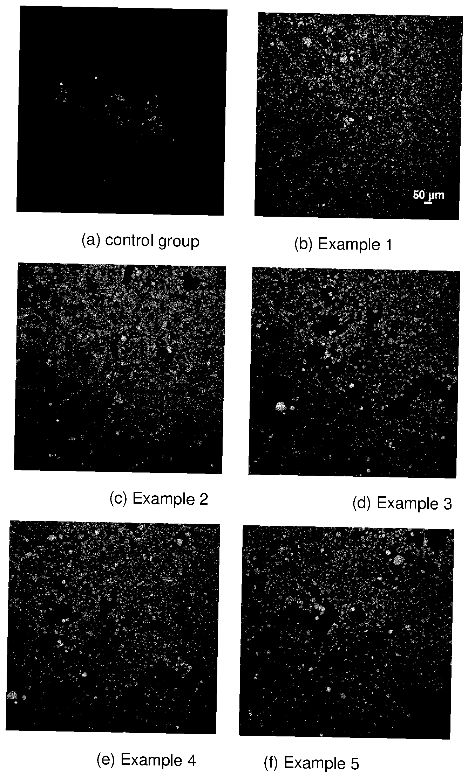

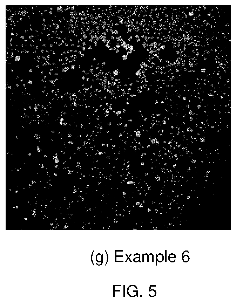

Fluorescence microscopy or flow cytometry was used to observe and record the number of cells on the surface of the valves. Specifically, endothelial cells were incubated at a density of 1.5 * 10⁴ cell/orifice into a 24-orifice cell culture plate with sample valves (prepared according to Examples 1 to 6, with a glutaraldehyde cross-linked valve unmodified with CD34 as control group) at the bottom. After incubating for 1 h, the unabsorbed cells were discarded, cell culture medium (DMEM medium) was added to continue culturing for 24 h, and then the culture medium was discarded and PBS was used to wash the valves for 3 times. After FDA staining for 15 minutes, washing with PBS 5 times, a fluorescent microscope was used to take pictures for observation. The valve with unmodified CD34 antibody was used as a contrast experiment. The cells on the pictures were counted by software such as IPP or imaged or manually. Alternatively, after the cells have been cultured for a period of time, they are digested with trypsin and counted manually under a phase-contrast microscope with a hemocytometer. The test results are shown in FIG. 5, which shows that the cross-linked valves modified by CD34 have good endothelial cell proliferation, with high specificity and capture ability, and are very suitable for cell endothelialization.

### Cytotoxicity

The cytotoxicity of the prepared antibody valves was observed by MTT method. Specifically, (1) cell culture: endothelial cells were cultured and sorted, and respectively incubated at 5000 cells per orifice into a 48-orifice plate where the valve materials prepared according to Examples 1 to 6 were placed, and cell culture medium (DMEM medium) was added to each orifice and incubated for 0 hour, 24 hours, 48 hours, 72 hours, with three replicate orifices for each group; (2) coloring: after taking out from the incubator, 500 µl cell culture medium and 50 µl MTT solution were added to each orifice (5 mg/ml), and incubated in an incubator at 37 °C for 4 hours; the cell culture medium was carefully aspirated, 500 µl DMSO was added and shaken fully for 10 minutes to fully dissolve the crystals; (3) colorimetry: 100 µl solution was taken out from each orifice and placed in a 96-orifice plate for detection; with a wavelength of 490 nm, and the control orifice as baseline, the absorbance values of the orifices were measured on the microplate reader; the cell growth curve was drawn with the time as the abscissa and the absorbance value as the ordinate. The test results are shown in Table 2, which shows that the cross-linked valves modified with CD34 have no cytotoxicity, with good biocompatibility.

**Table 2**

| Example No. | cell survival / % |
|---|---|
| Example 1-24 h | 87.11±7.92 |
| Example 1-48 h | 86.47±7.71 |
| Example 1-72 h | 86.14±5.44 |
| Example 2-24 h | 84.49±5.19 |
| Example 2-48 h | 84.21±3.58 |
| Example 2-72 h | 85.76±7.94 |
| Example 3-24 h | 86.88±4.92 |
| Example 3-48 h | 84.97±7.56 |
| Example 3-72 h | 84.77±8.44 |
| Example 4-24 h | 89.71±5.24 |
| Example 4-48 h | 85.47±6.82 |
| Example 4-72 h | 84.33±7.14 |
| Example 5-24 h | 87.67±5.22 |
| Example 5-48 h | 86.72±7.14 |
| Example 5-72 h | 84.55±8.20 |
| Example 6-24 h | 88.17±6.19 |
| Example 6-48 h | 82.72±4.22 |
| Example 6-72 h | 82.09±6.72 |

### Washing resistance of the antibody coating

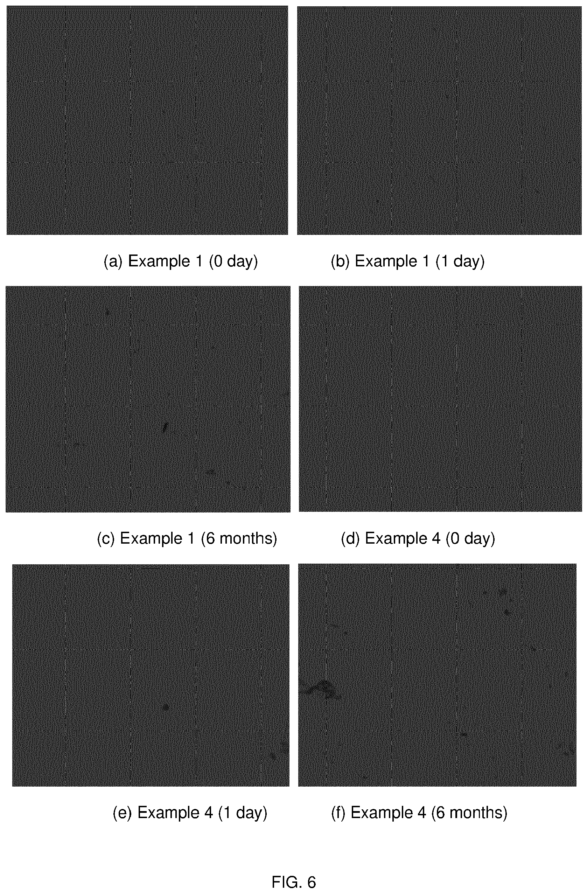

Fluorescence method or immunofluorescence method was used to observe the change of fluorescence intensity on the surface of the valve. Specifically, the antibody-modified dry valves prepared according to Examples 1 and 4 were placed into a test tube with PBS buffer solution in a shaker, at a constant temperature of 37 °C, at rotational speed of 100 r/min. After 1 day, the valves were taken out and the distribution of residual antibody on the surfaces of the valves was detected. The antibody-modified dry valves were placed in a sterilized container, sealed and preserved in a refrigerator at 4 °C for 6 months. Accordingly, the washing resistance of the antibody valves preserved for 6 months was tested. The test results are shown in FIG. 6, which shows that the cross-linked valves grafted with CD34 have strong washing resistance and CD34 can be remained on the valve surface for a long time.

### Long-term stability of the antibody coating

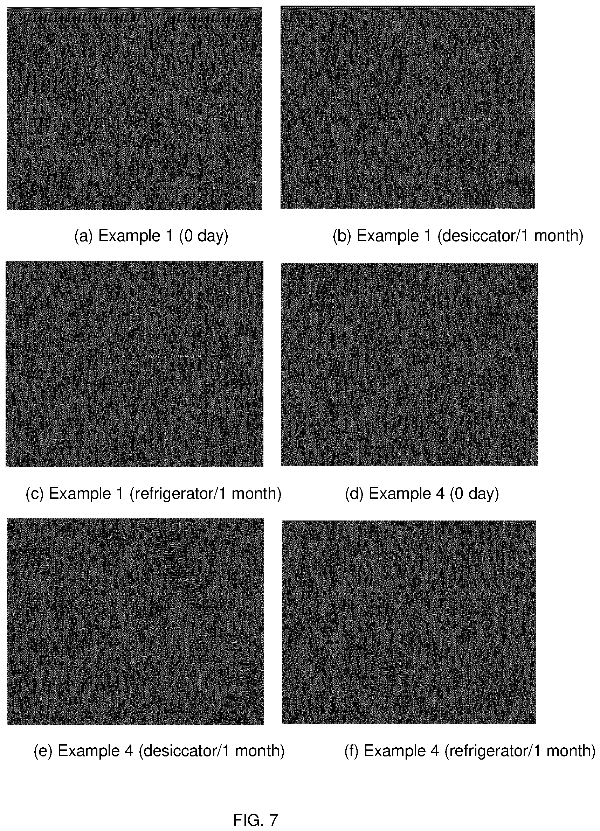

Fluorescence immunoassay was used to observe the changes of fluorescence intensity on the surface of the valve. Specifically, several pieces of prepared antibody-modified dry valves prepared according to Examples 1 and 4 were placed into sterilized containers respectively, and numbered sequentially. (1) 6 pieces of dry valves were put in a desiccator and preserved at room temperature for one month; (2) 6 pieces of dry valves were put in a refrigerator at 4 °C for one month. Immunofluorescence was performed by fluorescence immunoassay, and fluorescent photographs were taken to examine the activity of the antibody coating. The test results are shown in FIG. 7, which shows that the valves prepared here have long-term antibody stability.

The above-described embodiments only illustrate several embodiments of the present disclosure, and the description thereof is specific and detail, but should not be construed as limiting the scope of the patent disclosure. It should be noted that, for those of ordinary skill in the art, several modifications and improvements can be made without departing from the concept of the present disclosure, all of which fall into the protection scope of the present disclosure. Therefore, the protection scope of the present application should be based on the appended claims.

## Claims

1. A pro-endothelialization biological material, comprising a biological material and an endothelial growth factor loaded on the biological material.

2. The biological material according to claim 1, wherein the endothelial growth factor has a loading amount of: 0.1 to 20 µg of the endothelial growth factor on a surface of the biological material per surface area of 1 cm².

3. The biological material according to claim 1, wherein the biological material is a glutaraldehyde cross-linked biological material.

4. The biological material according to claim 1, wherein the endothelial growth factor is CD34 antibody, CD133 antibody or vascular endothelial growth factor (VEGF).

5. The biological material according to claim 1, wherein the endothelial growth factor is loaded on a surface of the biological material by chemical grafting or physical coating.

6. A preparation method for a pro-endothelialization biological material, comprising:
exposing a glutaraldehyde cross-linked biological material after removing free glutaraldehyde into a solution containing an endothelial growth factor for reaction, wherein the solution has a pH value of neutrality or weak alkalinity.

7. The preparation method according to claim 6, wherein in the solution containing the endothelial growth factor, the endothelial growth factor has a concentration ranging from 1 to 50 ug/ml.

8. The preparation method according to claim 7, wherein the solution containing the endothelialization growth factor further contains a polyamino substance; the polyamino substance is at least one of polyethylene glycol ammonia, aminopropylene glycol, amino acid and polypeptide; and the polyamino substance has a concentration ranging from 1 to 50 ug/ml.

9. The preparation method according to claim 6, wherein the solution containing the endothelial growth factor contains a solvent being PBS buffer, phosphate buffer or Tris-hydrochloric acid buffer, with a pH value ranging from 7 to 8.

10. The preparation method according to claim 6, comprising stopping the reaction when the endothelial growth factor on a surface of the pro-endothelialization biological material reaches a content ranging from 0.1 to 20 ug/cm².

11. The preparation method according to claim 6, wherein the reaction time ranges from 1 to 6 hours; and the reaction temperature ranges from 0 to 37 °C.

12. The preparation method according to claim 6, wherein during the reaction, the glutaraldehyde cross-linked biological material is in dynamic contact or static contact with the solution containing the endothelial growth factor.

13. The preparation method according to claim 6, further comprising exposing the glutaraldehyde cross-linked biological material after the reaction into a softening agent solution.

14. The preparation method according to claim 13, wherein the softening agent solution is a mixed solution of glycerin and polyethylene glycol or pure glycerin; and the glycerin has a volume percentage ranging from 20% to 100%.

15. The preparation method according to claim 13, further comprising dehydrating or drying the glutaraldehyde cross-linked biological material treated with the softening agent after removing free glycerin.

16. A preparation method for a pro-endothelialization biological material, comprising:
exposing a glutaraldehyde cross-linked biological material after removing free glutaraldehyde into a solution containing a capping substance and a solution containing an endothelial growth factor in sequence, wherein the capping substance is a substance that blocks residual aldehyde groups in the glutaraldehyde cross-linked biological material.

17. The preparation method according to claim 16, wherein the capping substance contains at least one group capable of reacting with the residual aldehyde group in the glutaraldehyde cross-linked biological material.

18. The preparation method according to claim 17, wherein the group for reacting with the residual aldehyde group is an amino group; and the solution containing the capping substance has a pH value of neutrality or weak alkalinity.

19. The preparation method according to claim 18, wherein the capping substance is a polyamino substance.

20. The preparation method according to claim 19, wherein the capping substance is at least one of polyethylene glycol ammonia, aminopropylene glycol, amino acid and polypeptide.

21. The preparation method according to claim 16, wherein in the solution containing the capping substance, the capping substance has a mass percent concentration ranging from 0.1% to 5%.

22. The preparation method according to claim 16, wherein the solution containing the capping substance contains a solvent being PBS buffer, phosphate buffer or Tris-hydrochloric acid buffer, with a pH value ranging from 7 to 8.

23. The preparation method according to claim 16, comprising stopping exposing the glutaraldehyde cross-linked biological material in the solution containing the capping substance when the residual aldehyde group amount of the glutaraldehyde cross-linked biological material is below a detectable value.

24. The preparation method according to claim 23, wherein the exposure time ranges from 1 to 6 hours.

25. The preparation method according to claim 16, wherein the capping substance is a reductant for reducing the residual aldehyde groups in the glutaraldehyde cross-linked biological material.

26. The preparation method according to claim 25, wherein the reductant is sodium borohydride or potassium borohydride.

27. The preparation method according to claim 16, wherein the endothelial growth factor is CD34 antibody, CD133 antibody or vascular endothelial growth factor (VEGF).

28. The preparation method according to claim 16, wherein a concentration of the endothelial growth factor in the solution containing the endothelial growth factor and the exposure time in the solution containing the endothelial growth factor are selected such that after finishing the preparation, the endothelial growth factor on a surface of the glutaraldehyde cross-linked biological material reaches a content ranging from 0.1 to 20 ug/cm².

29. The preparation method according to claim 16, the solution containing the endothelial growth factor contains a solvent being PBS buffer, phosphate buffer or Tris-hydrochloric acid buffer; and the endothelial growth factor has a concentration ranging from 1 to 50 ug/ml.

30. The preparation method according to claim 29, wherein the exposure time in the solution containing the endothelial growth factor ranges from 1 to 6 hours; and the reaction temperature ranges from 0 to 37 °C.

31. The preparation method according to claim 16, wherein the solution containing the endothelial growth factor has a pH value of neutrality.

32. The preparation method according to claim 16, comprising exposing the glutaraldehyde cross-linked biological material into the solution containing the capping substance and the solution containing the endothelial growth factor by dynamic contact or static contact.

33. The preparation method according to claim 16, further comprising exposing the glutaraldehyde cross-linked biological material after the reaction into a softening agent solution.

34. The preparation method according to claim 33, wherein the softening agent solution is a mixed solution of glycerin and polyethylene glycol or pure glycerin; and the glycerin has a volume percentage ranging from 20% to 100%.

35. The preparation method according to claim 33, further comprising dehydrating or drying the glutaraldehyde cross-linked biological material treated with the softening agent after removing free glycerin.

36. A pro-endothelialization biological material prepared by the preparation method of any one of claims 6 to 35.

37. A pro-endothelialization heart valve, comprising a cylindrical stent and a valve provided in the stent, wherein the valve is the pro-endothelialization biological material according to any one of claims 1 to 5 and 36.

38. A preparation method for a pro-endothelialization heart valve, the heart valve comprising a stent and a valve sewn on the stent, the valve being a glutaraldehyde cross-linked biological material, comprising:
treating the heart valve according to the method defined in any one of claims 6 to 35.

39. A preparation method for a pro-endothelialization heart valve, the heart valve comprising a stent and a valve, the valve being a glutaraldehyde cross-linked biological material, comprising:
treating the heart valve according to the method defined in any one of claims 6 to 35, and
sewing the valve on the stent.

40. A pro-endothelialization heart valve prepared by the preparation method of claim 38 or 39.

41. An interventional system, comprising a heart valve and a delivery catheter, the heart valve being configured to be delivered by the delivery catheter, wherein the heart valve is the pro-endothelialization heart valve according to any one of claims 37 or 40.
